(19) Europäisches Patentamt / European Patent Office / Office européen des brevets

(11) **EP 4 252 812 A1**

(12) **EUROPEAN PATENT APPLICATION**

(43) Date of publication:
**04.10.2023 Bulletin 2023/40**

(21) Application number: **22169786.5**

(22) Date of filing: **25.04.2022**

(51) International Patent Classification (IPC):
**A61M 16/10** $^{(2006.01)}$    **A61M 16/12** $^{(2006.01)}$
**A61M 16/06** $^{(2006.01)}$    **A61M 16/00** $^{(2006.01)}$
**A61M 16/20** $^{(2006.01)}$

(52) Cooperative Patent Classification (CPC):
**A61M 16/1005; A61M 16/026; A61M 16/0677;**
**A61M 16/125; A61M 16/204;** A61M 16/0066;
A61M 2016/0027; A61M 2016/0039;
A61M 2016/1025; A61M 2202/0208;
A61M 2205/3334; A61M 2205/3344; A61M 2205/50;
A61M 2230/205

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **30.03.2022 US 202263325196 P**

(71) Applicant: **Koninklijke Philips N.V.**
**5656 AG Eindhoven (NL)**

(72) Inventors:
• **BOU JAWDE, Samer**
 **Eindhoven (NL)**
• **DE GRAAF, Pascal**
 **Eindhoven (NL)**
• **BRANS, Harold Johannes Antonius**
 **Eindhoven (NL)**

(74) Representative: **Philips Intellectual Property &**
**Standards**
**High Tech Campus 52**
**5656 AG Eindhoven (NL)**

(54) **HIGH FLOW NASAL THERAPY SYSTEM AND METHOD**

(57)     A therapy system comprises a nasal cannula patient interface configured to deliver gas to a nasal cavity of a patient. A delivery system is used to delivering oxygen-enriched breathing gas, comprising air and enrichment oxygen, to the patient interface. A pressure sensor arrangement enables measurement or estimation of a pressure at the nasal cavity. A controller for is used to control the delivery system to deliver a target total flow and a target oxygen percentage, or to achieve a target SpO2 level, and to regulate the pressure. In one aspect, the pressure is regulated between first and second pressure levels during each breath, to provide bi-level positive airway pressure control. In another aspect, the delivery of enrichment oxygen is ended at a time which is at or before the end of an inhalation phase. Thus, in both aspects, there is pressure regulation of a high flow nasal therapy system and with control of the system in synchronism with the breathing cycle of the patient.

$$0 \leq t < T_x \qquad\qquad T_x \leq t < T_E + T_I$$
$$F_T^{i+1} = \alpha_i F_T^i \qquad\qquad F_T^{i+1} = F_D^{i+1} = \alpha_i F_T^i$$
$$F_D^{i+1} = \alpha_i F_D^i \qquad\qquad F_{O_2} = 0$$
$$F_{O_2}^{i+1} = \alpha_i F_{O_2}^i$$

FIG. 2

EP 4 252 812 A1

**Description**

FIELD OF THE INVENTION

This invention relates to High Flow Nasal Therapy (NFNT).

BACKGROUND OF THE INVENTION

HFNT provides high flows (compared to conventional flows) to patients through a nasal cannula. The basic idea is an extension to traditional oxygen support. Oxygen therapy typically provides only oxygen at low rates that usually range from 1 to 15 L/min. The oxygen is delivered via a so-called patient interface, which may comprise a nasal cannula, a face mask, a venturi mask, or a reservoir bag mask.

HFNT instead has two high pressure sources, for an air flow and for an oxygen flow. In combination, these high flows can reach a total of 60 L/min and more.

HFNT therapy is delivered through a device that has two main control functions, comprising the total rate of gas flow and the percentage of oxygen being delivered. The oxygen support is provided because HFNT was initially introduced as an elevated therapy for adults already on oxygen therapy (at normal flow rates) and for patients who are most likely hypoxemic or suffer from hypoxemic respiratory failure.

The basic device includes an air/oxygen blender and a heated humidifier to make sure the air is delivered at the appropriate temperature and humidity to the patient, mainly for comfort. With the blender, the fraction of inspired oxygen (FiO2) is for example controllable to range from 21% to 100%. The HFNT device is reported as offering several potential benefits, including:

> maintenance of a constant FiO2;
> generation of a positive end-expiratory pressure (PEEP) and increased end-expiratory lung volume;
> reduction of the anatomical dead space;
> improvement of mucociliary clearance; and
> reduction in the breathing frequency and work of breathing

Additionally, only requiring a nasal cannula instead of a mask means that HFNT provides much more comfort to the patients and allows them to perform more naturally their daily activities (e.g. eating, drinking, etc.).

Unfortunately, the primary use of HFNT is limited to stable hypoxemic patients, which restricts it to a limited patient population and also mainly restricts it to in-hospital usage. HFNT does not provide control over the pressure to allow for therapies that benefit other patients, e.g. hypercapnic patients.

The invention is based on the recognition that the potential of HFNT therapy is not fully utilized, such as the ability to provide dead space washout and CO2 clearance and a reduction in work of breathing.

US 2019/02011647 discloses a nasal therapy system which maintains a desired flow rate as well as pressure.

SUMMARY OF THE INVENTION

The invention is defined by the claims.

According to a first aspect of the invention, there is provided a therapy system, comprising:

> a nasal cannula patient interface configured to deliver gas to a nasal cavity of a patient;
> a delivery system for delivering oxygen-enriched breathing gas, comprising air and enrichment oxygen, to the patient interface;
> a pressure sensor arrangement to enable measurement or estimation of a pressure at the nasal cavity; and
> a controller for controlling the delivery system, wherein the controller is adapted to operate the delivery system to deliver a target total flow and a target oxygen percentage or to achieve a target SpO2 level, and to regulate the pressure between first and second pressure levels during each breath, to provide bi-level positive airway pressure control.

The first pressure level is an IPAP pressure and the second pressure level is an EPAP pressure.

The invention provides a HFNT therapy system (i.e. with oxygen delivery and air delivery to a nasal cannula) which is enhanced to provide control of the pressure support provided across the breathing cycle.

The pressure control is achieved by continuously sensing a pressure value (or sensing other parameters from which pressure can be derived), for example, at the nostrils, within the cannula, inside the mouth, or at the device just before the cannula. The delivery system is then controlled to obtain a set target pressure for example by altering the air flow (i.e. the gas delivery flow rate) or to obtain a target SpO2 level.

The control of the pressure support is achieved across the breathing cycle i.e. inhalation and exhalation. This enables the system to provide better therapy not only for hypoxemic patients but also to provide the benefits of comfort and simplicity of HFNT to other patient populations such as COPD and OSA patients, both of which have much higher prevalence and dominance in the population (in comparison to stable hypoxemic patients).

By providing this additional control over the parameters of HFNT, the therapy may be extended to provide a reliable treatment both at home and in hospital. For example, if the amount of pressure support (e.g. PEEP) is controlled then the device can be utilized at home for COPD or OSA patients instead of CPAP. This will provide the pressure support of a CPAP system, and in particular a BiPAP system, but with the advantage of the comfort of the HFNT approach. More importantly, as COPD is a major respiratory disease and patients are often treated through ventilators at home, this will greatly extend the patient population that can use HFNT.

The pressure is for example controlled by adapting flow rates.

The controller is for example adapted to end the delivery of enrichment oxygen by the delivery system at a time which is at or before the end of an inhalation phase.

Delivering oxygen during exhalation does not contribute to gas exchange, so oxygen can instead be conserved. Delivering oxygen near the end of inhalation may also not be as useful as at the start of inhalation.

According to a second aspect of the invention, there is provided a therapy system comprising:

    a nasal cannula patient interface configured to deliver gas to a nasal cavity of a patient;
    a delivery system for delivering oxygen-enriched breathing gas, comprising air and enrichment oxygen, to the patient interface;
    a pressure sensor arrangement to enable measurement or estimation of a pressure at the nasal cavity; and
    a controller for controlling the delivery system, wherein the controller is adapted to operate the delivery system to deliver a target total flow and a target oxygen percentage or to achieve a target SpO2 level, and to regulate the pressure,
    wherein the controller is adapted to end the delivery of enrichment oxygen by the delivery system at a time which is at or before the end of an inhalation phase.

This second aspect ends the delivery of oxygen as explained above. It may be applied to single level or bi-level pressure control.

The controller is for example adapted to regulate the pressure between first and second pressure levels during each breath, to provide bi-level positive airway pressure control.

Thus, the two features of bi-level pressure control and ending oxygen enrichment may be used individually or in combination.

The controller is for example adapted to increase the flow of air by the delivery system when the delivery of oxygen is ended. Thus, the overall flow is maintained, and hence the pressure control remains effective.

The system for example comprises a breath sensor for detecting the inhalation and exhalation phases of the breathing of the patient. This enables the pressure regulation, and the delivery of enrichment oxygen, to be timed relative to the breathing cycle.

The pressure sensor arrangement comprises one or more of:

    a pressure sensor at the nasal cavity;
    a flow sensor for measuring flow to the nasal cavity;
    a pressure sensor for measuring a pressure at the delivery system.

Thus, various measures of pressure and flow may be used to measure, or enable estimation of, the nasal cannula pressure. The sensed parameters may be combined with data relating to the flow resistance of the various components of the system.

The controller is for example adapted to set a maximum flow. This provides a safety measure.

The controller is for example controllable selectively to operate the system in one or more additional modes of operation, comprising:

    a first additional mode of operation wherein the controller is adapted to operate the delivery system to deliver a target total flow and a target oxygen percentage without pressure regulation; and
    a second additional mode of operation in which the delivery system is controlled to deliver a target pressure without oxygen enrichment.

The first additional mode is the standard HFNT approach without pressure support.

The second additional mode provides pressure support but without using the oxygen enrichment functionality. This is for example suitable for patients who are not hypoxemic but need pressure support (CPAP or BiPAP). The control is then simplified.

The invention also provides, in connection with the first aspect, a computer implemented method of controlling a therapy system comprising a nasal cannula patient interface configured to deliver gas to a nasal cavity of a patient, a delivery system for delivering oxygen-enriched breathing gas, comprising air and enrichment oxygen, to the patient interface, wherein the method comprises:

controlling the delivery system to deliver a target total flow and a target oxygen percentage, and to regulate the pressure between first and second pressure levels during each breath, to provide bi-level positive airway pressure control.

The method may then comprise ending the delivery of enrichment oxygen at a time which is at or before the end of an inhalation phase.

The invention also provides, in connection with the second aspect, a computer implemented method of controlling a therapy system comprising a nasal cannula patient interface configured to deliver gas to a nasal cavity of a patient, a delivery system for delivering oxygen-enriched breathing gas, comprising air and enrichment oxygen, to the patient interface, wherein the method comprises:

controlling the delivery system to deliver a target total flow and a target oxygen percentage, and to regulate the pressure; and
ending the delivery of enrichment oxygen by the delivery system at a time which is at or before the end of an inhalation phase.

The method may comprise increasing the flow of air by the delivery system when the delivery of oxygen is ended.

The methods may each also comprise selectively operating the system in one or more additional modes of operation, comprising:

a first additional mode of operation wherein the delivery system delivers a target total flow and a target oxygen percentage without pressure regulation; and
a second additional mode of operation wherein the delivery system delivers a target pressure without oxygen enrichment.

The invention also provides computer program comprising computer program code which is adapted, when said program is run on a computer, to implement the methods defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a therapy system for high flow nasal therapy;
Figure 2 shows the operating parameters of the system of Figure 1;
Figure 3 shows pressure waveforms for the operation of the system of Figure 1;
Figure 4 shows how the system of Figure 1 may be used in a simplified mode of operation to provide only pressure support; and
Figure 5 shows the corresponding waveforms for the mode of operation of Figure 4.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a therapy system comprises a nasal cannula patient interface configured to deliver gas to a nasal cavity of a patient. A delivery system is used to deliver oxygen-enriched breathing gas, comprising air and enrichment oxygen, to the patient interface. A pressure sensor arrangement enables measurement or estimation of a pressure at the nasal cavity. A controller for is used to control the delivery system to deliver a target total flow and a target oxygen percentage, or to achieve a target SpO2 level, and to regulate the pressure. In one aspect, the pressure is regulated between first and second pressure levels during each breath, to provide bi-level positive airway pressure control. In another aspect, the delivery of enrichment oxygen is ended at a time which is at or before the end of an inhalation phase. Thus, in both aspects, there is pressure regulation of a high flow nasal therapy system and with control of the system in synchronism with the breathing cycle of the patient.

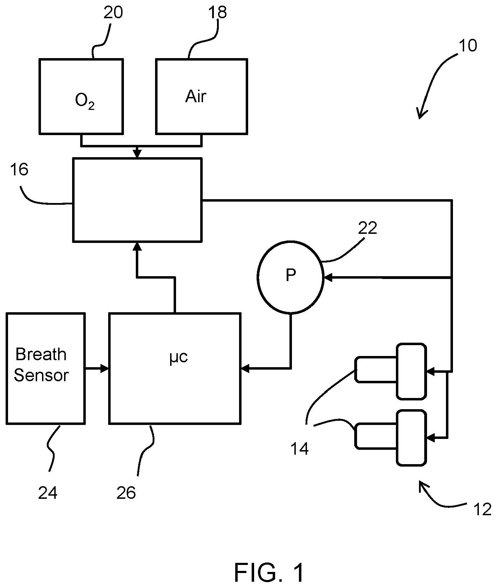

Figure 1 shows a therapy system 10 for high flow nasal therapy in schematic form.

The therapy system 10 comprises a nasal cannula patient interface 12 configured to deliver gas to a nasal cavity of a patient. The patient interface comprises a pair of nose prongs 14.

A delivery system 16 is provided for delivering oxygen-enriched breathing gas, comprising ambient air 18 and enrichment oxygen 20, to the patient interface 12. The delivery system for example comprises a flow pump (i.e. a blower) and a valve arrangement to control the coupling of ambient air and/or enrichment oxygen to the patient interface. It thus functions as an air/oxygen blender. The delivery system may also provide heating and humidification and/or dehumidification, not shown.

A pressure sensor arrangement 22 is provided to enable measurement or estimation of a pressure at the nasal cavity. It may comprise a pressure sensor, but other parameters such as flow rate may be monitored from which a pressure estimation may be derived.

A breath sensor 24 provides a breath sensor signal which enables the timing of the different phases (inhalation, exhalation) of the breathing cycle to be determined.

A controller 26 controls the delivery system. In particular, it controls the delivery system to control pressure levels and/or enrichment oxygen levels at variable levels over time within the timing of each individual breathing cycle. In one aspect, a target total flow and a target oxygen percentage are provided (i.e. typical HFNT parameters) but additionally the delivery pressure is regulated between first and second pressure levels during each breath, to provide bi-level positive airway pressure control. In another aspect, the controller is adapted to end the delivery of enrichment oxygen by the delivery system at a time which is at or before the end of an inhalation phase. The flow of air by the delivery system is for example increased when the delivery of oxygen is ended to maintain a desired flow rate. This reduces the amount of oxygen needed for HFNT therapy. The two approaches may be combined as part of a single therapy.

In addition to the new therapy approaches outlined above, the system may also be controllable to deliver existing conventional therapies, such as a first additional mode of operation with delivery of a target total flow and a target oxygen percentage without pressure regulation (i.e. conventional HFNT) and a second additional mode of operation in which the delivery system is controlled to deliver a target pressure without oxygen enrichment (i.e. conventional CPAP or bi-level PAP therapy). HFNT is conventionally always used with oxygen.

Currently, HFNT only controls the total delivered flow and the fraction of inspired oxygen (FiO2).

In one aspect, the invention is based on the recognition that the pressure resulting from HFNT can be used to provide pressure support and it can be controlled within the time duration of each breath, to enable bi-level pressure control. The pressure value which results from the control of flow depends on several factors but mainly the status of the mouth (open versus closed) and the total flow rate. A closed mouth and higher flow rates provide higher pressure levels.

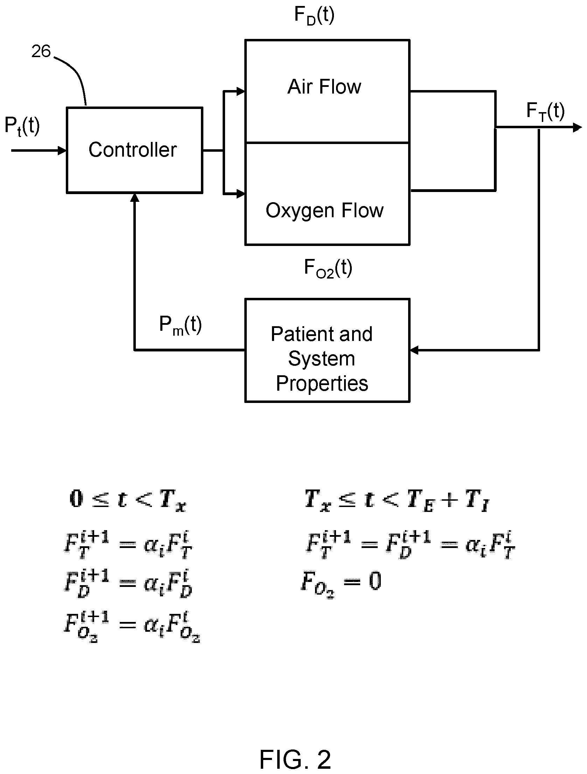

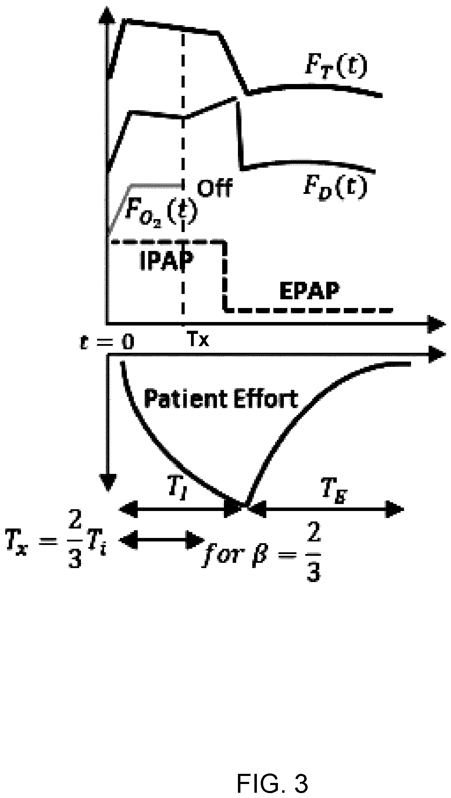

Figure 2 shows the operating parameters of the system and Figure 3 shows corresponding pressure waveforms.

In a conventional HFNT system, the controlled variables are the total flow (i.e. the combined air and enrichment oxygen flow to the patient) and the FiO2. The dependent variables are the air flow (i.e. the air flow to the patient interface without the additional oxygen flow) and the oxygen flow. Note that the air flow could be sourced from compressed air (common in a hospital setting) or simply from the room with the aid of a blower (common in home settings). Regardless of the source, the same concepts apply. The oxygen flow comes from a compressed source in both cases.

In the first aspect of the invention, the controlled variables are target pressures, in particular the inspiratory PAP and the expiratory PAP to provide bi-level pressure control, the FiO2 and an upper flow limit. The dependent variables are the air flow, the oxygen flow, total flow (oxygen + air flow) and the measured bi-level pressure values.

Figure 2 shows the air flow as $F_D(t)$ and the oxygen flow as $F_{O2}(t)$ which combine to give the total flow $F_T(t)$.

The controller 26 receives a target pressure Pt(t) over time. A feedback system determines a measured pressure $P_m(t)$ which is used by the controller to set the air flow and oxygen flow.

By using the control approach shown in Figure 2, a HFNT system can behave additionally as a BiPAP device. A user may set two target pressures, the IPAP and the EPAP. For safety, the user can also set a maximum flow limit that the device should not exceed. Alternatively, this limit can be a built-in value over which the user has no control but is determined by the manufacturer.

The controller receives the measured pressure $P_m(t)$ and adjusts both the air flow and oxygen flow to achieve the target pressure and FiO2. The measured pressure will depend on the system properties (e.g. interface, cannula resistance) as well as the patient characteristics (e.g. respiratory resistance, breathing cycle, mouth open, mouth closed etc.).

The pressure could be a measured pressure value at the level of the nasal prongs or an estimated pressure at the nasal prongs through modelling the system behavior (e.g. estimating pressure at the nasal prongs by having the device pressure, flow, and the system properties related to the resistance of the cannula).

Figure 2 includes equations showing that the flow levels $F_T$, $F_D$ and $F_{O_2}$ are controlled in an iterative way, wherein each is multiplied by a factor $\alpha_i$ at each iteration i.

The equations also show a second aspect, wherein after time $T_x$ (which is a fraction of the way through the inhalation phase of each breath cycle), the enrichment oxygen flow $F_{O_2}$ is reduced to zero but the total flow is kept the same and hence fully provided by the air flow.

The parameter $\alpha$ may be set by the manufacturer or by the user through an advanced user setting. It captures the change (increase or decrease) between the current flow delivered by the device and the new flow that will help achieve the target pressure. There could be several methods to set $\alpha$, at each iteration step (i).

One possible simple equation is given by:

$$\alpha(i) = \alpha_i = 1 - \frac{P_m - P_t}{P_t} \qquad \alpha_{min} \leq \alpha_i \leq \alpha_{max}$$

Where $P_m$, $P_t$, $\alpha_{min}$, and $\alpha_{max}$ represent the measured pressure, target pressure, minimum $\alpha$ and maximum $\alpha$ values.

For illustration, it is assumed that the flow is 30L/min, the target pressure is 10 cmH2O (10 cmH2O =9.8 Pa), $\alpha_{max}$ is 1.2 and $\alpha_{min}$ is 0.8.

The measured pressure may be assumed to be 8 cmH2O (7.8 Pa) and thus less than the target pressure. In this case, $\alpha$ is equal to exactly $\alpha_{max}$ [1-(8-10)/10]. The device will increase the total flow by 20% or by 1.2 to 36 L/min (30* 1.2).

The new measured pressure increases to 11 cmH2O (10.8Pa) and now is larger than the target pressure. The new value of $\alpha$ is now 0.9 [1-(11-10)/10] which falls within the range. The new flow will become 32.4 L/min (36*0.9). Now the measured pressure is 10.5 cmH20 (10.3 Pa) and is getting closer to target pressure as the process continues. Furthermore, as explained above, it is possible to limit the delivery of oxygen to conserve it as well as efficiently using it. Delivering oxygen during patient exhalation does not contribute to gas exchange. Also, delivering oxygen towards the end of inhalation might not be as useful as oxygen delivered at the beginning of inhalation. Thus, it is possible to set a time $T_x$ (measured from the onset of inhalation, patient effort, or $t$ = 0) that could be equal to or less than the inhalation time $T_i$ ($T_x = \beta T_i$ where $0 < \beta < 1$) to achieve that objective. The value of $\beta$ could be set by the manufacturer, or selected by the therapist based on need or observation such as for example based on monitoring SpO2 values.

After the time $T_x$ has passed, the oxygen delivered flow is turned off ($F_{O_2}$ = 0) and the loss in the total flow $(F_T)$ is compensated with an increase by the air flow $(F_D)$. Figure 3 shows an illustration how the various flows (oxygen, device, and total) will vary across a patient effort curve for BiPAP pressure control.

The oxygen flow stops at $T_x$ and the air flow increases to compensate, maintaining a stable total flow. Additionally, during inhalation, the total flow required to maintain the required pressure level of the bi-level control is larger than during exhalation due to the patient effort.

While delivering oxygen flow has been an essential element in HFNT, the ability to provide pressure support by itself is useful enough to be used in patients who are not hypoxemic but in need of pressure support (i.e. CPAP or BiPAP). Thus, this decouples the current classical notion regarding the coupling between oxygen support and HFNT allowing the latter to have wider applications.

Increasing the pressure will further expand and recruit the lung units allowing more gas exchange to occur. This will then increase the amount of oxygen in the blood and thus the SpO2 level. Thus, since the level of SpO2 depends on the pressure which itself depends on the flow by varying the flow, the system can also be used to control the SpO2 of patients.

The incremental change in pressure explained above (using value $\alpha$) applies to a baseline $\alpha$ value that can achieve the designated pressure(s). In other words, rather than abruptly changing flow to reach the desired target, this mechanism comfortably, safely, and gradually changes the flow rates to reach the desired overall pressure level and flow settings. The adaptation using the value $\alpha$ could occur during the initial setup across a breath or across a couple of breath in order to reach an estimated baseline. However, once set-up, and during the breath cycle itself, the switching between flow levels to achieve and maintain the IPAP and EPAP pressures is as near to instantaneous as possible (as can be seen in the traces for $F_D(t)$ and $F_T(t)$ in Figure 3).

Thus, once the system has reached a required overall operating point with an estimated baseline $\alpha$, the pressure levels basically toggle between the same two values (one for inhalation and one for exhalation) at each successive breath by instantaneous flow variation.

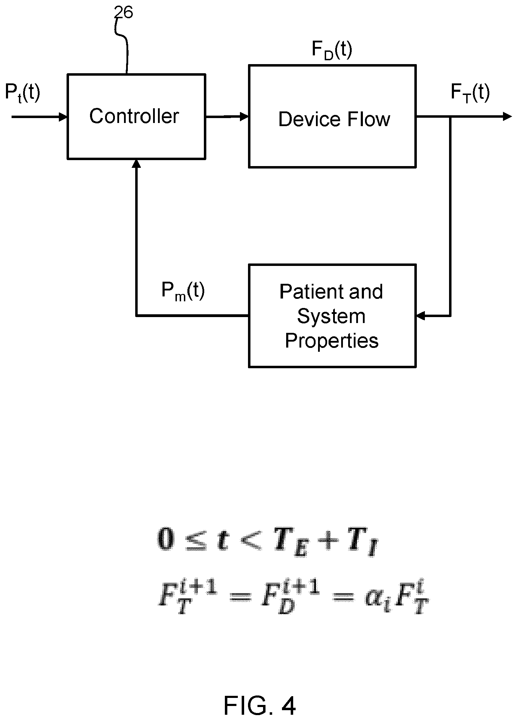

Figure 4 shows how the system may be used to provide only pressure support. In this case, the controller 26 only controls the air flow $F_D(t)$ which is thus equal to the total flow $F_T(t)$. This simplifies the process of the controller since it now only has one source of flow.

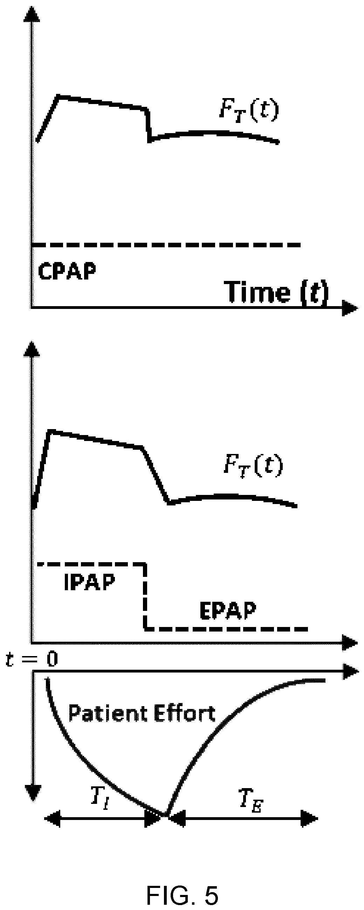

Figure 5 shows the corresponding waveforms.

The control approach of Figures 2 and 3 makes use of a time value Tx which is a fraction of the inhalation time duration Ti. Thus, the inhalation time duration needs to be known at the beginning of the inhalation period, or the patient effort timing needs to be known. This is the purpose of the breath sensor 24.

The inhalation time duration Ti could be continuously estimated from several previous cycles (e.g. from the previous 50 breaths). The inhalation time duration can be obtained by detecting the transition from inhalation to exhalation. One way to detect this transition is by analysis of the measured target pressure; a sudden increase in target pressure will indicate exhalation since the patient effort is now in recoil and less flow device is needed to achieve target pressure.

Thus, the breath sensor 24 may in fact be a function implemented by the controller 26 from the received pressure signal. However, additional sensors may also be used. For example, sensing operating conditions of various circuit elements (e.g. blower) of the delivery system (e.g. power, current, voltage...) may be used to detect changes which arise between inhalation and exhalation.

In particular, the patient breathing during HFNT is driven by their own respiratory effort, as the device delivers high flow through the nasal cannula. During inhalation, the blower of the delivery system is supported by the patient flow as the patient attempts to breath in. During exhalation, the opposite occurs. The blower is resisted by the patient flow as the patient attempts to exhale. This will change the rotary speed of the blower. Electronics in the blower module will correct for this and change the power until the revolution speed is at the correct level. Thus, there is a decrease in blower power during patient inhalation and an increase during exhalation. Therefore, by tracking the power consumed by the blower across time, the patient's respiratory effort can be indirectly tracked.

The inhalation/exhalation (IE) state can then be determined by looking at the pattern of the blower power variation. The transition from the exhalation phase to the inhalation phase is visible by a drop in power consumption. This is independent with respect to both the flow level of the HFNT device and the breathing effort of the patient. In other words, by observing the change at any given flow and patient effort, the increase and decrease in power consumption detects IE transition.

By knowing the transition from the exhalation phase to the inhalation phase, the repeating cycle (respiratory rate, RR) is also known. When the blower power variation is measured in time, the RR can statistically be monitored to detect shortness of breath (dyspnea).

The amplitude of the blower power consumption curve also reveals information about the level of effort the patient applies during breathing. The lower the amplitude, the lower the effort. On the other hand, the shape of the curve shows if the breathing effort is balanced and fits the normal pattern.

Surrogates of blower power may instead be used to detect the IE state, such as the current output of the controller to the delivery system or monitoring the change in rotational speed. Any components (and elements) in the HFNT device that are influenced by the IE breathing cycle of the patient can be monitored, and are possible surrogates for the blower power.

The system could also be used to detect when the nasal cannula is not properly detected or totally disconnected. In case the total flow delivered is constantly increasing (e.g. this can be detected if $\alpha$ is not changing and being set to the maximum value at every iteration for a considerable time (e.g. 5-10 minutes while the pressure measured is minimally changing), then a warning could be provided to the user to properly set the nasal cannula or put it on. In the case of no response or change in few minutes, then most probably the patient is not using the device and it could be turned off automatically.

To avoid any pressures beyond physiologically accepted levels, or pressures that could be high for specific patients (as determined by a patient's clinician for example), the device could have an additional pressure safety to avoid reaching this pressure. This could be done by reducing the flow or limiting the flow to the values that do not lead to the pressure being exceeded.

The therapy system may have an additional use mode, wherein SpO2 is measured. The therapist can then set a target SpO2 and provide a range of pressure values that are acceptable. The device can then achieve the required oxygen level by automatically finding the optimal pressure and optimal FiO2 (or oxygen flow). Blood oxygenation levels are dependent on both the PEEP level and the amount of FiO2. Thus, theoretically, there exists an optimal and safe point at a designated PEEP level and FiO2 level that achieves the required SpO2 while conserving oxygen.

This method avoids the need to have a specific target pressure and further saves oxygen consumption. In other words, from a baseline pressure value, and based on the measured SpO2, the pressure could be increased or decreased to achieve the target SpO2. To reach the required pressures, the same technique in finding the baseline pressure using $\alpha$ could be applied as explained above, so that reaching the optimal pressure values is gradual, safe, and comfortable.

In this mode (setting a desired SpO2 level and an IPAP/EPAP/CPAP range), the oxygen conservation also

occurs through optimizing $\beta$. In other words, at a fixed pressure, both the time and amount of oxygen flow effect and determine SpO2. For example, a short time of oxygen delivery at higher oxygen flow (higher FiO2) could achieve the same SpO2 as delivering oxygen for a longer time at a lower oxygen flow (lower FiO2). If the former, in total, delivers (i.e. consumes) less oxygen than the latter, then it is more efficient and preferred, and hence is automatically selected by the device. Thus, an internal mechanism will vary both the oxygen flow value and the oxygen delivery time to select the optimal set of these two parameters that achieve the target SpO2 at a given pressure range.

The application has wide use both at home and in hospitals/clinics. It is of particular interest for COPD and OSA patients and particularly mild to moderate cases in need of pressure support in addition to stable hypoxemic patients.

The description above refers to High Flow Nasal Therapy, HFNT. It is however noted other terms are also used to describe the same therapy or device, such as high flow nasal cannula (HFNC), nasal high flow (NHF) or high flow nasal oxygen (HFNO). The term HFNT is intended to cover all of these different therapies and devices.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A therapy system, comprising:

   a nasal cannula patient interface (12) configured to deliver gas to a nasal cavity of a patient;
   a delivery system (16) for delivering oxygen-enriched breathing gas, comprising air and enrichment oxygen, to the patient interface;
   a pressure sensor arrangement (22) to enable measurement or estimation of a pressure at the nasal cavity; and
   a controller (26) for controlling the delivery system, wherein the controller is adapted to operate the delivery system to deliver a target total flow and a target oxygen percentage or to achieve a target SpO2 level, and to regulate the pressure between first and second pressure levels during each breath, to provide bi-level positive airway pressure control.

2. The system of claim 1, wherein the controller is adapted to end the delivery of enrichment oxygen by the delivery system at a time which is at or before the end of an inhalation phase.

3. A therapy system, comprising:

   a nasal cannula patient interface (12) configured to deliver gas to a nasal cavity of a patient;
   a delivery system (16) for delivering oxygen-enriched breathing gas, comprising air and enrichment oxygen, to the patient interface;
   a pressure sensor arrangement (22) to enable measurement or estimation of a pressure at the nasal cavity; and
   a controller (26) for controlling the delivery system, wherein the controller is adapted to operate the delivery system to deliver a target total flow and a target oxygen percentage, and to regulate the pressure,
   wherein the controller is adapted to end the delivery of enrichment oxygen by the delivery system at a time which is at or before the end of an inhalation phase.

4. The system of claim 3, wherein the controller is adapted to regulate the pressure between first and second pressure levels during each breath, to provide bi-level positive airway pressure control.

5. The system of claim 3 or 4, wherein the controller is adapted to increase the flow of air by the delivery system when the delivery of oxygen is ended.

**6.** The system of any one of claims 1 to 5, comprising a breath sensor (24) for detecting the inhalation and exhalation phases of the breathing of the patient.

**7.** The system of any one of claims 1 to 6, wherein the pressure sensor arrangement (22) comprises one or more of:

> a pressure sensor at the nasal cavity;
> a flow sensor for measuring flow to the nasal cavity;
> a pressure sensor for measuring a pressure at the delivery system.

**8.** The system of any one of claims 1 to 7, wherein the controller is adapted to set a maximum flow.

**9.** The system of any one of claims 1 to 7, wherein the controller is controllable selectively to operate the system in one or more additional modes of operation, comprising:

> a first additional mode of operation wherein the controller is adapted to operate the delivery system to deliver a target total flow and a target oxygen percentage without pressure regulation; and
> a second additional mode of operation in which the delivery system is controlled to deliver a target pressure without oxygen enrichment.

**10.** A computer implemented method of controlling a therapy system comprising a nasal cannula patient interface configured to deliver gas to a nasal cavity of a patient, a delivery system for delivering oxygen-enriched breathing gas, comprising air and enrichment oxygen, to the patient interface, wherein the method comprises:
controlling the delivery system to deliver a target total flow and a target oxygen percentage, and to regulate the pressure between first and second pressure levels during each breath, to provide bi-level positive airway pressure control.

**11.** The method of claim 10 comprising ending the delivery of enrichment oxygen at a time which is at or before the end of an inhalation phase.

**12.** A computer implemented method of controlling a therapy system comprising a nasal cannula patient interface configured to deliver gas to a nasal cavity of a patient, a delivery system for delivering oxygen-enriched breathing gas, comprising air and enrichment oxygen, to the patient interface, wherein the method comprises:

> controlling the delivery system to deliver a target total flow and a target oxygen percentage or to achieve a target SpO2 level, and to regulate the pressure; and
> ending the delivery of enrichment oxygen by the delivery system at a time which is at or before the end of an inhalation phase.

**13.** The method of claim 11 or 12, comprising increase the flow of air by the delivery system when the delivery of oxygen is ended.

**14.** The method of any one of claims 10 to 13, comprising selectively operating the system in one or more additional modes of operation, comprising:

> a first additional mode of operation wherein the delivery system delivers a target total flow and a target oxygen percentage without pressure regulation; and
> a second additional mode of operation wherein the delivery system delivers a target pressure without oxygen enrichment.

**15.** A computer program comprising computer program code which is adapted, when said program is run on a computer, to implement the method of any one of claims 8 to 10.

FIG. 1

$F_D(t)$

26

$P_t(t)$

Controller

Air Flow

Oxygen Flow

$F_T(t)$

$F_{O2}(t)$

$P_m(t)$

Patient and
System
Properties

$$0 \leq t < T_x \qquad\qquad T_x \leq t < T_E + T_I$$

$$F_T^{i+1} = \alpha_i F_T^i \qquad\qquad F_T^{i+1} = F_D^{i+1} = \alpha_i F_T^i$$

$$F_D^{i+1} = \alpha_i F_D^i \qquad\qquad F_{O_2} = 0$$

$$F_{O_2}^{i+1} = \alpha_i F_{O_2}^i$$

FIG. 2

$$T_x = \frac{2}{3} T_i \quad \text{for } \beta = \frac{2}{3}$$

FIG. 3

$$0 \leq t < T_E + T_I$$

$$F_T^{i+1} = F_D^{i+1} = \alpha_i F_T^i$$

FIG. 4

FIG. 5

Europäisches
Patentamt

European
Patent Office

Office européen
des brevets

## EUROPEAN SEARCH REPORT

Application Number

EP 22 16 9786

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/175857 A1 (WHITING DAVID ROBIN [NZ] ET AL) 13 June 2019 (2019-06-13) | 1-9,15 | INV. A61M16/10 |
| A | * figures 1-4 * <br> * paragraph [0038] - paragraph [0066] * | 10-14 | A61M16/12 A61M16/06 A61M16/00 |
| X | US 2019/160242 A1 (SCHWAIBOLD MATTHIAS [DE] ET AL) 30 May 2019 (2019-05-30) | 1-9,15 | A61M16/20 |
| A | * figures 1-6 * <br> * paragraph [0010] - paragraph [0066] * | 10-14 | |
| X | US 10 799 663 B1 (AIRES MEDICAL LLC [US]) 13 October 2020 (2020-10-13) | 1-9,15 | |
| A | * figures 1I-M * <br> * paragraph [0051] - paragraph [0089] * | 10-14 | |
| X | US 2021/001075 A1 (ODDO NICHOLAS LEONARD [US] ET AL) 7 January 2021 (2021-01-07) | 1-9,15 | |
| A | * figures 1-9 * <br> * paragraph [0027] - paragraph [0050] * | 10-14 | |
| X | WO 2017/096428 A1 (RESMED LTD [AU]) 15 June 2017 (2017-06-15) | 1-9,15 | TECHNICAL FIELDS SEARCHED (IPC) |
| A | * figures 1-12 * <br> * paragraph [0157] - paragraph [0225] * | 10-14 | A61M |

The present search report has been drawn up for all claims

| Place of search | Date of completion of the search | Examiner |
|---|---|---|
| Munich | 13 October 2022 | Liess, Helmar |

EPO FORM 1503 03.82 (P04C01)

## ANNEX TO THE EUROPEAN SEARCH REPORT
## ON EUROPEAN PATENT APPLICATION NO.

EP 22 16 9786

This annex lists the patent family members relating to the patent documents cited in the above-mentioned European search report.
The members are as contained in the European Patent Office EDP file on
The European Patent Office is in no way liable for these particulars which are merely given for the purpose of information.

13-10-2022

| Patent document cited in search report | | Publication date | Patent family member(s) | | Publication date |
|---|---|---|---|---|---|
| US 2019175857 | A1 | 13-06-2019 | AU 2018274993 A1 | | 27-06-2019 |
| | | | US 2019175857 A1 | | 13-06-2019 |
| US 2019160242 | A1 | 30-05-2019 | CN 109966605 A | | 05-07-2019 |
| | | | DE 102018009288 A1 | | 06-06-2019 |
| | | | EP 3492131 A1 | | 05-06-2019 |
| | | | EP 3950031 A1 | | 09-02-2022 |
| | | | US 2019160242 A1 | | 30-05-2019 |
| US 10799663 | B1 | 13-10-2020 | US 10874817 B1 | | 29-12-2020 |
| | | | US 2020179638 A1 | | 11-06-2020 |
| | | | US 2020306486 A1 | | 01-10-2020 |
| | | | US 2021196918 A1 | | 01-07-2021 |
| US 2021001075 | A1 | 07-01-2021 | NONE | | |
| WO 2017096428 | A1 | 15-06-2017 | EP 3386576 A1 | | 17-10-2018 |
| | | | EP 3884986 A1 | | 29-09-2021 |
| | | | JP 7069017 B2 | | 17-05-2022 |
| | | | JP 2019501700 A | | 24-01-2019 |
| | | | JP 2022119780 A | | 17-08-2022 |
| | | | NZ 743113 A | | 26-03-2021 |
| | | | US 2019111226 A1 | | 18-04-2019 |
| | | | US 2022203058 A1 | | 30-06-2022 |
| | | | WO 2017096428 A1 | | 15-06-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 201902011647 A